(19) Europäisches Patentamt
European Patent Office
Office européen des brevets

(11) Publication number: **0 127 325**
**B1**

(12) # EUROPEAN PATENT SPECIFICATION

(45) Date of publication of patent specification: **22.02.89**

(51) Int. Cl.$^4$: **C 07 D 303/02,**
C 07 D 303/38, B 01 F 17/00,
C 09 B 67/00

(21) Application number: **84302861.4**

(22) Date of filing: **27.04.84**

(54) **Dispersing agent.**

(30) Priority: **24.05.83 GB 8314325**

(43) Date of publication of application:
**05.12.84 Bulletin 84/49**

(45) Publication of the grant of the patent:
**22.02.89 Bulletin 89/08**

(84) Designated Contracting States:
**CH DE FR GB IT LI NL SE**

(56) References cited:
**GB-A- 790 314**
**GB-A-1 342 746**
**US-A-2 745 846**

**CHEMICAL ABSTRACTS, vol. 91, no. 2, 09-07-1979, page 94, nr. 6764c, Columbus, Ohio, US; S.D. WAGH et al.:"Reduction of epoxidized acetylated castor oil and epoxidized acetylated ricinoleic acid by hydrazine hydrate" & J. Oil Technol. Assoc. India 1978 10(4), 154-5**

(73) Proprietor: **IMPERIAL CHEMICAL INDUSTRIES PLC**
**Imperial Chemical House Millbank**
**London SW1P 3JF (GB)**

(72) Inventor: **Schofield, John David**
**Old Oak House 482 Holcombe Road**
**Greenmount Bury Lancashire BL8 4HB (GB)**

(74) Representative: **Pugsley, Roger Graham et al**
**IMPERIAL CHEMICAL INDUSTRIES PLC Legal Department: Patents PO Box 6 Bessemer Road**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 127 325 B1**

## Description

This specification describes an invention relating to a compound suitable for dispersing a solid in an organic liquid and to a composition containing the compound.

A number of very effective dispersing agents for a wide range of solids, including pigments, dyestuffs, oil drilling muds etc., in essentially non-polar organic liquids, especially hydrocarbons and chlorinated hydrocarbons, are known from UK Patent Numbers 1,342,746, 1,373,660 and 2,001,083. These agents contain mono- or poly-ester residues derived from an aliphatic hydroxy carboxylic acid containing at least 9 carbon atoms with at least 4 carbon atoms separating the carboxyl and hydroxyl groups, a preferred acid being hydroxystearic acid. These agents can, however, have a deleterious effect on the properties of paint films when dispersions containing them are converted into paints. It has now been found that agents of this type are improved, particularly for use in thermosetting paints, if they contain epoxy groups.

An ester of epoxidised ricinoleic acid with a $C_{1-6}$-primary aliphatic alcohol is disclosed as a plasticiser for vinyl choride polymers in GB—A—790,314 and the reduction of an epoxidised acetylated ricinoleic acid with hydrazine is disclosed in Chemical Abstracts (Abstract No. 91—6764c, 1979).

According to the present invention there is provided a compound of the formula:

$$B—([O—A—CO]_n—J)_m \qquad I$$

wherein

A is alkylene or alkenylene containing from 8 to 25 carbon atoms with at least 4 carbon atoms between the oxygen atom and the carbonyl group;

m is 1 or 2;

n is an integer from 1 to 19;

B is $Y(—CO—)_m$ in which:

when m = 1, Y is alkyl, alkenyl, alkapolyenyl containing from 8 to 25 carbon atoms or arylalkyl or arylalkenyl containing up to 50 carbon atoms, and

when m = 2, Y is alkylene, alkenylene or alkapolyenylene containing up to 50 carbon atoms;

and

J is —OM in which M is H, $NH_4$ or a metal;

or

J is —ZR in which:

Z is —N(Q)—L— or —O—L— in which:

Q is H or $C_{1-25}$-alkyl; and

L is $C_{2-6}$-alkylene or $C_{2-6}$-hydroxyalkylene; and

R is an amino group or the salt thereof with an acid;

or

R is a sulphonic acid, phosphonic acid, sulphate or phosphate group, or the salt thereof with a metal or an amine;

provided that at least 30% of the total number of groups represented by A, B and J carry an epoxy group. Preferably at least 30% of the groups represented by A carry an epoxy group.

Where $n > 1$ the groups represented by A in the polyester chain $[O—A—CO]_n$ may be the same (i.e. it is a homopolyester) or different (i.e. it is a copolyester) and as few as 30% of the groups represented by A in the polyester chain need carry epoxy groups. It is however preferred that at least 50% and more preferably at least 70% of the groups represented by A carry an epoxy group. The group represented by A may carry up to three epoxy groups but preferably carries a single epoxy group. It is especially preferred that at least 30% of the groups represented by A are 8,9-epoxy-11-n-heptyl-unadec-1,11-ylene. It is also especially preferred that n is from 1 to 10.

In addition to carrying epoxy groups, the groups represented by A may carry other substituents which do not confer water-solubility on the molecule, such as halogen and alkoxy.

The group $Y(—CO—)_m$ represented by B in Formula I may be considered as the residue of an esterifiable carboxylic acid Y—COOH in which the group Y, when m = 2, is alkylene, alkenylene or alkapolyenylene containing up to 50 carbon atoms and, when m = 1, is alkyl, alkenyl or alkapolyenyl, containing from 8 to 25 carbon atoms or arylalkyl or arylalkenyl containing up to 50 carbon atoms. The aryl group present in Y is preferably phenyl. The aliphatic groups represented by Y may carry substituents that do not confer water solubility on the molecule, such as hydroxy, halogen, amino and alkoxy, but are preferably unsubstituted. The group represented by Y may also carry up to three epoxy groups. When B represents a group $Y(—CO—)_m$ it is preferred that m is 1.

A preferred polyester chain of the formula, $[O—A—CO]_n$, is derivable from an unsaturated aliphatic hydroxy carboxylic acid containing from 8 to 25 carbon atoms having at least 4 carbon atoms between the hydroxy and carboxyl groups and a carbon-carbon double bond. In the compound of Formula I, containing such a polyester chain, it is preferred that at least 30%, and more preferably 50%, of these carbon-carbon double bonds are epoxidised. An example of a suitable unsaturated hydroxy carboxylic acid is ricinoleic acid. In a polyester derived from pure ricinoleic acid the groups represented by A and Y are the same and Y carries a free hydroxy group. However, in their normal commercial forms, hydroxy carboxylic acids often

contain small quantities of similar acids which are free from hydroxy groups. These can terminate the polyester chains and form the terminal group, represented by B, when B = Y[—CO—]$_m$ in which Y is a simple hydrocarbon chain free from hydroxy groups. Examples of suitable acids from which the end group Y(—CO—)$_m$ is derivable, by reaction with a terminal hydroxy group on the polyester chain, are stearic acid, oleic acid, linoleic acid, linolenic acid, phenylacetic acid, methoxyacetic acid and abietic acid in which any double bonds may be previously or subsequently epoxidised.

In the group —OM represented by J in Formula I, M is preferably H or an alkali or alkaline earth metal, especially calcium.

Examples of the group represented by Q are methyl, ethyl, n-propyl, n-butyl and octadecyl and of the group represented by L are ethylene, trimethylene, tetramethylene, hexamethylene and 2-hydroxytrimethylene.

The amino group represented by R may be a primary, secondary or tertiary amino group or a substituted ammonium group and is preferably of the formula:

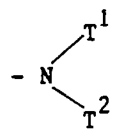

or

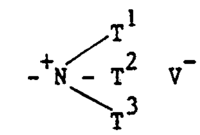

wherein $T^1$, $T^2$ and $T^3$ are each independently H, alkyl, or cycloalkyl, in which the alkyl group preferably contains up to 25 carbon atoms and the cycloalkyl preferably contains from 4 to 8 carbon atoms and each may be substituted by non-polar groups and $V^-$ is a coloured or colourless anion. Examples of the groups represented by $T^1$ to $T^3$ are methyl, ethyl, n-propyl, n-butyl, octadecyl, 2-hydroxyethyl and cyclohexyl. The anion, $V^-$, can be from any inorganic or coloured or colourless organic acid, such as HCl, $H_2SO_4$, $CH_3COOH$, $C_2H_5COOH$, HCOOH, $CH_3SO_3H$, $C_6H_5SO_3H$, $C_6H_5COOH$ or an organic dye containing a —$SO_3H$ or —COOH group, especially an azo, anthraquinone or phthalocyanine dye containing a —$SO_3H$ or —COOH group, such as are described in the Third Edition of the Colour Index (1971).

Alternatively the amine group, R, may be a polyalkylene imine as disclosed in UK 2,001,083 or such a polyalkylene imine carrying polyester side chains. The compound of Formula I above in which J comprises a polyalkylene imine is similar to the polyester-polyalkylene-imine dispersing agent described and claimed in UK 2,001,083 except that one or more, and preferably all, of the polyester side chains contain epoxy groups. A preferred polyalkylene imine is a polyethylene imine having a molecular weight in the range from 500 to 100,000.

Where R represents a sulphonic acid, phosphonic acid, sulphate or phosphate group or the salt thereof with a metal or an amine the cation may be a hydrogen ion, a metal ion, an ammonium ion or a substituted ammonium ion and examples of suitable cations are $H^+$, $Na^+$, $K^+$, $Ca^{2+}$, $NH_4^+$, $N(CH_3)_4^+$ and $NH(CH_3)_4^+$.

The compound of Formula I can be prepared by the processes described in UK Patent Nos. 1,342,746, 1,373,660 and 2,001,083, provided that at least one of the reactants contains at least one carbon-carbon double bond, followed by epoxidation of the carbon-carbon double bond or bonds. According to a further feature of the present invention there is provided a process for the preparation of a compound according to Formula I which comprises reacting an epoxidixing agent with a compound of the formula:

$$B\text{—}([O\text{—}A\text{—}CO]_n\text{—}J)_m \qquad \text{III}$$

wherein B, A and J are as previously defined provided that they are free from epoxy groups and at least 30% of the total number of these groups contain a carbon-carbon double bond. Such a process is particularly suitable for the preparation of compounds of Formula I which are made by esterification or amidification because of the sensitivity of epoxy groups to free amino and hydroxy groups. Where the compound contains a quaternary ammonium group, quaternisation may precede or follow the epoxidation.

If the compound of Formula I contains groups, other than epoxidisable double bonds, which are unstable to epoxidising agents it is preferable to perform the epoxidation at an earlier stage and according to another feature of the present invention there is provided a process for the preparation of a compound according to Formula I which comprises reacting a compound of the formula B(—W)$_m$ with a compound of the formula:

$$HO\text{—}A\text{—}CO\text{—}(O\text{—}A\text{—}CO)_{n-1}\text{—}J \qquad \text{IV}$$

wherein W is a group reactive with hydroxy groups, and A, B, m and n have the same meanings as in Formula I and J is a monovalent aliphatic group.

The epoxidising agent may be any epoxidising agent in general use. It is conventional to use a peroxy acid and especially an organic peroxy acid as epoxidising agent. The organic peroxy acid may be in the aliphatic or aromatic series and may carry polar substituents. Suitable peroxy acids include performic acid, peracetic acid, perbenzoic acid, m-chloroperbenzoic acid, perpropionic acid, trifluoroperacetic acid, monoperoxyphthalic acid and p-nitroperbenzoic acid.

The peroxy acid may be used directly or generated in situ. It may be generated in situ by reaction of the

corresponding carboxylic acid with a suitable oxidising agent, such as hydrogen peroxide. It is often desirable to promote the in situ formation of the peroxy acid by the addition of an acid catalyst, such as sulphuric acid. Otherwise the acid catalysis may be provided by an immobilised acid catalyst such as an ion-exchange resin containing sulphonic acid groups.

Epoxidation may be performed in the absence or the presence of a solvent. Suitable solvents include chloroform, dichloromethane, benzene, toluene, xylene, 1,2-dichloroethane and 1,1,1-trichloroethane.

Other epoxidising agents which may be suitable are peroxyimidic acids, generated in situ from an alkyl nitrile and hydrogen peroxide, ethoxyperformic acid, generated in situ from ethoxychloroformate and hydrogen peroxide and hydrogen peroxide itself in the presence of tungstic or molybdic acid at a pH between 4 and 6. The epoxidation may be performed using an electrochemical oxidation method.

Processes for the preparation of the compound of Formula III are analogous to those given in the aforementioned patents, using either an unsaturated hydroxycarboxylic acid in place of the saturated hydroxycarboxylic acid disclosed in said patents for the preparation of the mono- or poly-ester residue followed by epoxidisation, or an epoxidised hydroxycarboxylic acid. The disclosures of UK Patent Specification Nos. 1,342,746, 1,373,660 and 2,001,083 are incorporated into the present specification by reference.

The compound according to Formula I is suitable for the dispersion of a solid in an organic liquid and according to a further feature of the present invention there is provided a dispersible composition of a solid and a compound according to Formula I.

It is preferred that the amount of the compound of Formula I, hereinafter referred to as the dispersing agent, present in the composition is from 1% and 50%, and more preferably from 2% to 30%, by weight, based on the amount of solid.

The composition is suitable for the preparation of a dispersion of the solid in an organic liquid and according to a further feature of the present invention there is provided a dispersion of a solid in an organic liquid containing a compound according to Formula I. The dispersion preferably contains from 5% to 90%, by weight, of the solid, based on the total weight of the dispersion, the precise quantity depending on the nature of the solid and the relative densities of the solid and the organic liquid. For example, dispersions of organic solids preferably contain from 15% to 60% by weight of solid whereas dispersions of inorganic solids preferably contain from 40% to 90% of the solid, based on the total weight of the dispersion.

The organic liquid in which the solid is dispersed may be any inert organic liquid in which the dispersing agent is at least partially soluble at ambient temperatures and which is stable under the subsequent conditions of usage of the dispersion. If desired mixtures of organic liquids may be used. Preferred organic liquids are aromatic hydrocarbons and aliphatic and aromatic halogenated hydrocarbons such as benzene, toluene, xylene, chlorobenzene, carbon tetrachloride and perchloroethylene. Other organic liquids which can be used are esters, such as propyl acetate, aliphatic alcohols such as n-butanol and ketones such as methylbutylketone and particularly mixtures of these with aromatic hydrocarbons, such as 4:1 xylene:n-butanol, which is widely used in the preparation of thermosetting paints. In general the organic liquid or mixture of liquid selected will depend on the end-use for the dispersion.

The dispersed solid is preferably an inorganic or organic pigment from any of the recognised classes of pigments described, for example, in the Third Edition of the Colours Index (1971) and subsequent revisions and supplements, under the chapter headed "Pigments". Other preferred solids are fillers and extenders such as talc, kaolin, barytes and chalk.

Examples of inorganic pigments are titanium dioxide, zinc oxide, Prussion blue, cadmium sulphide, iron oxides, carbon black, vermillion and ultramarine. Further examples of inorganic pigments are the chrome pigments including chromates, molybdates and mixed chromates/sulphates of lead, zinc, barium and calcium and the various mixtures and modifications thereof which are commercially available as greenish-yellow to red pigments under the names primrose, lemon, middle, orange, scarlet and red chromes.

Examples of organic pigments are those from the azo, disazo, condensed disazo, thioindigo, indanthrone, isoindanthrone, anthraquinone, anthranthrone, isodibenzanthrone, triphendioxazine, phthalocyanine, especially copper phthalocyanine and its nuclear halogenated derivatives, and quinacridone series, and lakes of acid, basic and mordant dyes. Preferred organic pigments are copper phthalocyanines, monoazos, disazos, indanthrones and quinacridones.

The dispersion may also contain other ingredients such as are normally added to improve the properties and performance of dispersions. Examples of such other ingredients are fluidising agents, anti-sedimenting agents, preservatives against microbial growth and resins.

The present dispersions are compatible with the fluidising agents disclosed in UK Patent Specifications Nos 1,508,576 and 2,108,143 the contents of which are incorporated herein by reference. The fluidising agent described in UK 1,508,576 is a substituted ammonium salt of a coloured acid wherein there are from 19 to 60 carbon atoms in at least 3 chains attached to the N atom of the substituted ammonium ion. The fluidising agent described in UK 2,108,143 is a water-insoluble disazo compound comprising a central divalent group free from acidic and other ionic substituents linked, through azo groups, to two monovalent end groups, one end group being free from acidic and other ionic substituents and the other carrying a single substituted ammonium-acid salt group. The present dispersions incorporating such fluidising agents form a further feature of the present invention.

The dispersions of the invention can be obtained by any of the conventional methods for preparing

dispersions. Thus the solid, the organic liquid and the dispersing agent may be mixed together in any order and, where necessary, the mixture subjected to a mechanical treatment to reduce the particle size of the solid, for example by ball, gravel or bead milling until a stable dispersion is formed. The treatment is preferably continued until the mean diameter of the pigment particles is below 10 microns and more preferably below 5 microns. The composition of the solid and the compound of Formula I can be isolated by removal of the solvent, for example, by evaporation.

If the dispersing agent is used in the form of a salt it is not essential to take the preformed salt as this can be prepared in situ during the preparation of the dispersion by mixing together the solid, the organic liquid, the appropriate dispersing agent, in its free acid or base form, and either a base or acid, and subjecting the mixture to a mechanical grinding operation. It is not essential that the dispersing agent is converted completely into the salt form because in many cases the free acid or base form of the agent is equally effective in dispersing the solid.

An alternative process comprises reducing the particle size of the solid alone, or in admixture with the organic liquid or the dispersing agent alone followed by the addition of the other ingredients when the desired reduction in particle size has been achieved. Yet another process comprises adding an aqueous emulsion of a solution of the dispersing agent in an organic liquid to an aqueous slurry of the solid, following which the organic liquid and water are removed, by filtration and the solid mixture is dried to provide a dry composition of the solid and the dispersing agent. A dry composition of a solid and a compound of Formula I forms a further feature of the present invention. The dry composition can be subsequently incorporated into an organic liquid to give a dispersion. Where the solid is a pigment, such a dry composition can be formed by mixing the dispersing agent, preferably in an aqueous emulsion, with the solid during a late stage in the preparation of the pigment so that the dispersing agent forms an intimate mixture with the pigment.

The dispersing agent is particularly suitable for the dispersion of pigments destined for use in paints, especially thermosetting paints, because the agent is compatible with the resins used in such paints and does not adversely affect the properties of the paint film, such as hardness, gloss, transparency, etc. In this respect it is superior to the aforementioned known dispersing agents of similar structure which do not contain epoxy groups while retaining the powerful dispersing action of these known agents.

Paint systems in which the dispersing agent, and dispersions derived therefrom, are particularly suitable include alkyd/melamine-formaldehyde paints, alkyd/urea-formaldehyde paints, thermosetting acrylic/melamine-formaldehyde paints, vinyl-modified alkyd/melamine-formaldehyde paints and epoxy paints. Other thermosetting or stoving paints for which the dispersing agents and derived dispersions are suitable are those based upon combinations of oil-free alkyd resins, oil-free polyester resins, vinyl-modified alkyd resins, styrene-modified alkyd resins, rosin/phenolic modified alkyd resins and polymethacrylic ester-modified alkyd resins with crosslinkers or hardeners, such as melamine-formaldehyde, urea-formaldehyde, butylated melamine-formaldehyde, butylated urea-formaldehyde or hexamethoxymethylmelamine.

The invention is further illustrated by the following examples in which all parts and percentages are by weight unless otherwise indicated.

### Intermediate 1

A mixture of 3206 g of ricinoleic acid and 6 g of tetrabutyl titanate was stirred at 170—5°C under a stream of inert gas. The acid value of the reaction mixture was determined at intervals and after 17 hours, when the acid value was 34 mg KOH/g, reaction was stopped by cooling the reaction mixture to room temperature. The product is hereinafter called Intermediate 1.

### Intermediate 2

A mixture of 1276 g of Intermediate 1, 40.5 g of dimethylaminopropylamine and 104 g of toluene was stirred under reflux at 190°C using a Dean & Stark head to remove water. After two hours, when no further water was being removed, the toluene was removed by distillation under vacuum. The reaction product, hereinafter called Intermediate 2, had an acid value of 24.4 mg KOH/g and an equivalent weight, with respect to basic nitrogen, of 3459.

### Intermediate 3

To 980 g of Intermediate 2, stirring at 40°C, there were added 33.8 g of dimethylsulphate. An exotherm resulted, raising the temperature to 50°C and this was raised to 90° and maintained there for 1½ hours by the application of external heat. At the end of this period no free dimethyl sulphate could be detected in the reaction mass by gas-liquid chromatography. The product is hereinafter called Intermediate 3.

### Intermediate 4

A mixture of 350 g of ricinoleic acid, 100 g of oleic acid and 0.2 g of tetrabutyl titanate was stirred at 180—185°C under a stream of inert gas. The acid value of the reaction mixture was determined at intervals and, after 8½ hours, when the acid value was 70.3 mg, KOH/g, reaction was stopped by cooling the reaction mixture to room temperature. The product is hereinafter called Intermediate 4.

Intermediate 5

A mixture of 82.5 g of Intermediate 4 and 6.22 g of highly branched high molecular weight polyethyleneimine (sold under the trade name POLYMIN WATERFREE by BASF) was stirred at 145—155°C for 1½ hours. The reaction was then stopped by cooling the reaction mixture. The product, hereinafter called Intermediate 5, had an acid value of 35.8 mg KOH/g.

Intermediate 6

A mixture of 200 g ricinoleic acid, 87 g of oleyl alcohol and 0.3 g tetrabutyl titanate was stirred at 170—180°C under a stream of inert gas. The acid value was determined at intervals and after 23 hours when the acid value was 2 gm KOH/g, reaction was stopped by cooling the reaction mixture to room temperature. The product, hereinafter called Intermediate 6, had a hydroxyl value of 57 mg KOH/g.

Intermediate 7

Solution A was prepared by dissolving 3.8 g aminoethane sulphonic acid in a mixture of 2.5 g of a 48.6% aqueous solution of sodium hydroxide and 25 g water.

Solution B was prepared by mixing 50 g Intermediate 1 and 21.7 g toluene until the mixture was homogeneous.

Solution B was transferred to a flask equipped with a gas inlet tube, and a Dean and Stark water separator. Solution A was added to the flask over 1 minute while the contents were stirred rapidly. Stirring was continued while heating was applied, and initially all the water was removed in the Dean and Stark separator. Toluene was then distilled off until the temperature of the reaction mass rose to 180°C. The reaction mass was then stirred at this temperature for 5½ hours under a stream of inert gas, after which reaction was stopped by cooling the reaction mixture to room temperature. The reaction product, hereinafter called Intermediate 7, had an acid value of 5.9 mg KOH/g.

Intermediate 8

To a solution of 50 g of Intermediate 6 in 296 g chloroform there was added, over a period of one hour, a solution of 40 g of m-chloroperbenzoic acid in 592 g chloroform. An exotherm resulted raising the temperature to approximately 40°C. One hour after addition was complete, a test with dampened starch-iodide paper showed the presence of excess peroxy acid. 2½ hours after addition the same test showed the absence of peroxy acid. The reaction mixture was allowed to stand overnight, and any crystallised m-chlorobenzoic acid was filtered off. The chloroform solution was first washed with 200 g of 10% aqueous sodium sulphite solution, and then with 400 g of 5% sodium bicarbonate solution. Finally the chloroform solution was washed until free from alkali with several 800 g portions of water and the chloroform removed, by vacuum distillation, to leave the product, hereinafter called Intermediate 8.

Intermediate 6 had an iodine value of 91.3 g $I_2$/100 g while Intermediate 8 had an iodine value of 15.5 g $I_2$/100 g indicating that the m-chloroperbenzoic acid had epoxidised 83% of the double bonds present in Intermediate 6.

Example 1

To a solution of 50 g of Intermediate 1 in 296 g of chloroform there was added, over a period of one hour, a solution of 46 g of m-chloroperbenzoic acid in 660 g of chloroform. An exotherm resulted raising the temperature to 40°C. After a further two hours stirring, during which the temperature was allowed to fall to ambient, a further 4 g of m-chloroperbenzoic acid was added and stirring continued for 30 minutes. The reaction mixture was then checked for the absence of excess peroxy acid using dampened starch-iodide paper. It was then allowed to stand overnight and any crystallised m-chlorobenzoic acid was filtered off. After the addition of 200 g of 5% aqueous sodium sulphite solution with agitation, the mixture was allowed to stand and the chloroform layer separated from the aqueous layer. The product was worked up by washing the organic layer with 800 g 5% sodium bicarbonate solution and then with several 800 g portions of water until free from alkali. The chloroform was then removed by vacuum distillation leaving the product, hereinafter called Dispersing Agent 1.

Intermediate 1 had an iodine value of 94 g $I_2$/100 g while Dispersing Agent 1 had an iodine value of 15.8 g $I_2$/100 g indicating that the m-chloroperbenzoic acid had epoxidised 83.2% of the double bonds present in Intermediate 1.

Example 2

To a solution of 23 g of Intermediate 2 in 148 g of chloroform was added, over 30 minutes, a solution of 15 g of m-chloroperbenzoic acid in 220 g of chloroform. An exotherm raised the temperature to 35°C and after a further period of 2 hours, during which the temperature fell to ambient, a further 2 g of m-chloroperbenzoic acid was added. After a further 30 minutes of agitation and standing overnight the reaction mixture was tested for presence of peracid (a trace was found) using dampened starch-iodide paper. The product was worked up and isolated as in Example 1. It is hereinafter called Dispersing Agent 2.

Example 3

To a solution of 62.5 g of Intermediate 3 in 296 g of chloroform there was added, over 1 hour, a solution of 50 g of m-perbenzoic acid in 740 g of chloroform. An exotherm raised the temperature to 35°C and the mixture was agitated for a further 2 hours and allowed to stand overnight. A test with dampened starch-iodide paper showed the presence of some excess peracid. The product was worked up and isolated as in Example 1. It is hereinafter called Dispersing Agent 3.

Intermediate 3 had an iodine value of 90.5 g $I_2$/100 g and Dispersing Agent 3 had an iodine value of 7 g $I_2$/100 g. This indicates that the m-chlorobenzoic acid had epoxidised 92% of the double bonds present in Intermediate 3.

Example 4

To a solution of 50 g of Intermediate 4 in 296 g of chloroform was added over 1 hour a solution of 43 g m-chloroperbenzoic acid in 600 g chloroform. An exotherm raised the temperature to approximately 40°C. After agitation for a further hour, a test with dampened starch-iodide paper showed the absence of peroxy acid. The reaction mixture was allowed to stand overnight. The product was then worked up as in Example 1. It is hereinafter called Dispersing Agent 4. Intermediate 4 had an iodine value of 98.6 g $I_2$/100 g while Dispersing Agent 4 had an iodine value of 21.9 g $I_2$/100 g indicating that the m-chlorobenzoic acid had epoxidised 78% of the double bonds present in Intermediate 4.

Example 5

To a solution of 30 g of Intermediate 5 in 222 g chloroform was added over 30 minutes a solution of 25 g m-chloroperbenzoic acid in 370 g chloroform. An exotherm raised the temperature to approximately 35°C, and the colour of the reaction mixture lightened during the addition from a dark brown to a pale yellow. After agitation for a further twenty minutes, a test with dampened starch-iodide paper showed a trace of peroxy acid. The reaction mixture was allowed to stand overnight. The product was then worked up as in Example 1. It is hereinafter called Dispersing Agent 5. Intermediate 5 had an iodine value of 96.5 g $I_2$/100 g while Dispersing Agent 5 had an iodine value of 49.9 g $I_2$/100 g indicating that the m-chloroperbenzoic acid had epoxidised 48% of the double bonds present in Intermediate 5.

Example 6

To a solution of 16 g of Intermediate 7 in 104 g chloroform was added over 30 minutes a solution of 13 g of m-chloroperbenzoic acid in 192 g chloroform. The temperature was kept below 35°C during the addition. After allowing the reaction mixture to stand for 16 hours, a test with dampened starch-iodide paper showed no peroxy acid was present. The product was then worked up as in Example 1. It is hereinafter called Dispersing Agent 6. Intermediate 7 had an iodine value of 91 g $I_2$/100 g while Dispersing Agent 6 had an iodine value of 8.27 g $I_2$/100 g indicating that the m-chloroperbenzoic acid had epoxidised 91% of the double bonds present in Intermediate 7.

Example 7

To a solution of 26.6 g of Intermediate 1 in 148 g chloroform was added over 30 minutes a solution of 10 g m-chloroperbenzoic acid in 148 g chloroform. A slight exotherm raised the temperature by a few degrees centigrade. After agitation for a further two hours, a test with dampened starch-iodide paper showed the absence of peroxy acid. The product was then worked up as in Example 1. It is hereinafter called Dispersing Agent 7. Intermediate 1 had an iodine value of 94 g $I_2$/100 g while Dispersing Agent 7 had an iodine value of 57 g $I_2$/100 g indicating that the m-chloroperbenzoic acid had epoxidised 40% of the double bonds present in Intermediate 1.

Example 8

To a solution of 280 g of Intermediate 1 and 47 g of formic acid in 1000 g of dichloromethane stirred at 20—25°C there was added, over a period of 6 hours, 170 g of a 35% by weight solution of hydrogen peroxide. The reaction mixture was stirred at 20—25°C during a further 18 hours and then checked for the presence of excess peroxy acid using dampened starch-iodide paper. The excess peroxy acid was destroyed by the addition of 40% aqueous sodium hydrogen sulphite solution. To the mixture was added 400 g of a 1% solution of cetrimide. The mixture was allowed to stand for 1 hour and the dichloromethane layer was separated from the aqueous layer. The organic layer was washed to freedom from acid with several 400 g portions of water. The dichloromethane was removed by vacuum distillation leaving the product, hereinafter called Dispersing Agent 8.

Dispersing Agent 8 had an iodine value of 37.5 g $I_2$/100 g indicating that the performic acid formed in situ had epoxidised 60% of the double bonds present in Intermediate 1.

Example 9

In place of the 170 g of 35% solution of hydrogen peroxide used in Example 8 there were used 119 g of a 50% solution of hydrogen peroxide. The product, Dispersing Agent 9, had an iodine value of 27.9 g $I_2$/100 g indicating that the performic acid formed in situ had epoxidised 70% of the double bonds present in Intermediate 1.

Example 10

To a solution of 280 g of Intermediate 1 in 1000 g of dichloromethane stirred at 20—25°C was added 4 g of anhydrous sodium acetate followed, over a period of 5 hours, by 240 g of 38% peracetic acid. The reaction mixture was stirred at 20—25°C during a further 18 hours. The excess of peroxy acid was destroyed by the addition of 40% aqueous sodium hydrogen sulphite solution, when a test sample showed no coloration by spotting on to dampened starch-iodide paper. To the mixture was added 800 g of a 1% solution of centrimide. The mixture was allowed to stand for 1 hour and the dichloromethane layer was separated from the aqueous layer. The organic layer was washed to freedom from acid with several 800 g portions of water. The dichloromethane was removed by vacuum distillation leaving the product, hereinafter called Dispersing Agent 10.

Dispersing Agent 10 had an iodine value of 29.8 g $I_2$/100 g indicating that the peracetic acid had epoxidised 68% of the double bonds present in Intermediate 1.

Fluidising Agent 1

This is the product described as Agent B in UK 1508576.

Fluidising Agent 2

This is the product described in Example 1 of UK 2108143.

Fluidising Agent 3

This is the product described in Example 1 of UK 2108143.

Comparative Dispersing Agent A

This is poly(12-hydroxystearic acid) having an acid value of 35 mg KOH/g.

Comparative Dispersing Agent B

A mixture of 320 g of Comparative Dispersing Agent A, 10.2 g of dimethylaminopropylamine and 65 g of toluene was stirred under reflux using a Dean & Stark head to remove water of reaction. The temperature was then raised to 165°C and held there for 6 hours. Finally the toluene was removed by vacuum distillation.

Comparative Dispersing Agent C

To 200 g of Comparative Dispersing Agent B, stirring at 40°C, was added 7.1 g of dimethyl sulphate. An exotherm raised the temperature to 50°C and this was raised to 90°C by the application of external heating. At the end of 1 hour at 90°C no free dimethyl sulphate was detected in the product by gas-liquid chromatography.

Comparative Dispersing Agent D

This is polyricinoleic acid having an acid value of 34 mg KOH/g, and it is also referred to in this patent application as Intermediate 1.

Comparative Dispersing Agent E

A mixture of 335 g 12-hydroxystearic acid, 100 g stearic acid and 1.0 g tetrabutyl titanate was stirred at 170—180°C under a stream of inert gas. The acid value was determined at intervals and after 12 hours, when the acid value was 73 mg KOH/g, reaction was stopped by cooling the reaction mixture to room temperature. The product is hereinafter called Comparative Dispersing Agent E.

Examples 11 to 32

Twenty-two dispersions, having the formulations described in Table 1 were prepared by ball-milling the ingredients for 16 hours. All the resulting dispersions were fluid, deflocculated and with the pigment particles having a mean diameter below 5 microns.

In Table 1 the Pigments are identified by their shade and Colour Index (CI) number. The Dispersing Agents and Fluidising Agents are identified by DA and FA respectively followed by the number identified hereinbefore in the description of these agents.

Table 1

| Example | Pigment & Amount | Dispersing Agent & Amount | Fluidising Agent & Amount | Org Liq & Amount |
|---|---|---|---|---|
| 11 | White 6 7.0g | DA 1 0.35g | - | Xylene 2.65g |
| 12 | Red 104 6.0g | DA 2 0.6g | - | Xylene 3.4g |

| Example | Pigment & Amount | Dispersing Agent & Amount | Organic Liquid & Amount | |
|---|---|---|---|---|
| 13 | Red 101 6.0g | DA 2 0.6g | - | Xylene 3.4g |
| 14 | Green 7 3.0g | DA 3 0.6g | FA 1 0.3g | Xylene 6.1g |
| 15 | Black 7 3.0g | DA 3 0.6g | FA 1 0.3g | Xylene 6.1g |
| 16 | Yellow 1 3.0g | DA 3 0.6g | FA 2 0.3g | Xylene 6.1g |
| 17 | Red 4 3.0g | DA 3 0.6g | FA 3 0.3g | Xylene 6.1g |
| 18 | Yellow 34 7.5g | DA 5 0.37g | - | Xylene 2.13g |
| 19 | Blue 15:2 3.0g | DA 5 0.6g | FA 1 0.3g | Xylene 6.1g |
| 20 | Red 122 2.5g | DA 5 0.5g | - | Xylene 7.0g |
| 21 | Yellow 173 2.5g | DA 5 0.5g | - | Xylene 7.0g |
| 22 | Yellow 34 6.0g | DA 4 0.6g | - | Xylene 3.4g |
| 23 | Yellow 34 7.0g | DA 6 0.35g | - | Toluene 2.65g |
| 24 | Yellow 34 7.0g | DA 6 0.35g | 2-ethoxyethyl acetate 2.65 | |
| 25 | Yellow 34 7.0g | DA 6 0.35g | methyl-isobutyl ketone 2.65g | |
| 26 | Yellow 34 7.0g | DA 6 0.35g | butyl acetate 2.65 | |
| 27 | Yellow 34 7.0g | DA 6 0.35g | n-butanol 2.65g | |
| 28 | Yellow 34 5.0g | DA 6 0.25g | perchloroethylene 4.75 | |
| 29 | White 6 7.5g | DA 6 0.23g | Xylene 2.27g | |
| 30 | Yellow 34 7.0g | DA 7 0.35g | Xylene 2.65g | |
| 31 | Yellow 34 7.5g | DA 8 0.38g | Xylene 2.12g | |
| 32 | Yellow 34 7.5g | DA 10 0.38g | Xylene 2.12g | |

Compatibility Testing

In order to evaluate the compatibility of dispersing agents with various paint resin systems, the following experiments were performed.

An Alkyd/Melamine formaldehyde paint resin system

A stock solution containing 100 g PLASTOKYD C30—AX, 50 g BEETLE BE 615 and 50 g xylene was prepared. (PLASTOKYD is a registered trade mark of Cray Valley Products Ltd., and PLASTOKYD C30—AX is an alkyd resin comprising 42% phthalic anhydride, 40% fatty acid, 18% glycerol and 30% xylene. BEETLE is a registered trade mark of British Industrial Plastics and BEETLE BE 615 is an n-butylated melamine formaldehyde resin supplied as a 60% solution in n-butanol). This stock solution was divided into 4 g samples which were diluted with a further 2 g xylene. Dispersing agent was then added to, and dissolved in, these samples. Table 2 indicates the dispersing agents added. In each case they were added at levels of 0%, 1%, 2%, 5% and 10% calculated as weight on total resin solids weight. A portion of each sample was then poured on to a glass slide, dimension 7.5 cm by 2.5 cm. The quantity used was sufficient to give a film approximately 75 microns thick, on drying. The slides were air-dried for 30 minutes, then stoved for 30 minutes at 120°C. Complete compatibility is shown by a clear, transparent slide. Incompatibility is manifested in a cloudy, milky appearance to the slide. The slides were assessed on a 0 to 5 scale where 0 indicates complete transparency, and 5 indicates extreme milkiness. The results are reported in Table 2.

Table 2

| Dispersing Agent | % Dispersing Agent in film | | | | |
|---|---|---|---|---|---|
| | 0% | 1% | 2% | 5% | 10% |
| Comparative Disp.Agent A | 0 | 0 | 1 | 1–2 | 3 |
| B | 0 | 0 | 1 | 2 | 3 |
| C | 0 | 1 | 1–2 | 3 | 4 |
| D | 0 | 0 | 0 | 0–½ | 0–½ |
| Dispersing Agent 1 | 0 | 0 | 0 | 0–1 | 0–1 |
| 2 | 0 | 0 | 0 | 0 | 0 |
| 3 | 0 | 0 | 0 | 0 | 0 |
| 4 | 0 | 0 | 0 | 0 | 0 |
| 5 | 0 | 0 | 0 | 0 | 0 |
| 6 | 0 | 0 | 0 | 0 | 0–½ |
| 7 | 0 | 0 | 0 | 0 | 0–½ |
| 8 | 0 | 0 | 0 | 0 | 0 |

A Thermosetting Acrylic Paint System

A stock solution containing 187.5 g SYNOCRIL 826S, 125 g BEETLE BE 615 and 62.5 g xylene was prepared (SYNOCRIL is a registered trade mark of Cray Valley Products Ltd., and SYNOCRIL 826S is a hydroxyl acrylic resin supplied as a 60% solution in xylene/n-butanol). Glass slides of this resin system containing various dispersing agents at various levels were prepared in exactly the same way as in the previous experiment.

The slides were assessed on the same 0 to 5 scale, and the results are reported in Table 3.

Table 3

| Dispersing Agent | % Dispersing Agent in film | | | | |
|---|---|---|---|---|---|
| | 0% | 1% | 2% | 5% | 10% |
| Comparative Disp.Agent A | 0 | 1 | 1 | 3 | 4 |
| B | 0 | 0 | 1 | 3 | 5 |
| C | 0 | 0 | 1 | 1-2 | 3-4 |
| D | 0 | 0 | 0 | ½ | 1-2 |
| E | 0 | 0 | 0 | 0 | ½ |
| Dispersing Agent 1 | 0 | 0 | 0 | 0 | 0 |
| 2 | 0 | 0 | ½ | ½ | 1-2 |
| 3 | 0 | 0 | 0 | 0 | ½ |
| 4 | 0 | 0 | 0 | 0 | 0 |
| 5 | 0 | 0 | 0 | 0 | 0 |
| 6 | 0 | 0 | 0 | 0 | 0-½ |
| 7 | 0 | 0 | 0 | ½ | 1 |
| 8 | 0 | 0 | 0 | 0 | ½ |

An Epoxy Paint Resin System

A stock solution containing 107.1 g ARALDITE PZ 820, 35.7 g ARALDITE HZ 820, 34.3 g xylene, 5.7 g n-butanol and 17.2 g methyl isobutyl ketone was prepared. (ARALDITE is a registered trade mark of CIBA-GEIGY Plastics and Additives Co., ARALDITE PZ 820 is an epoxy resin supplied as a 70% solution in a blend of aromatic, hydroxylic and ketonic solvents, and ARALDITE HZ 820 is a polyaminoamide curing agent also supplied as a 70% solution in a blend of aromatic, hydroxylic and ketonic solvents). Glass slides of this resin system containing various dispersing agents at various levels were prepared in exactly the same way as the previous two experiments. The slides were assessed on the same 0 to 5 scale, and results are reported in Table 4.

Table 4

| Dispersing Agent | % Dispersing Agent in film | | | | |
|---|---|---|---|---|---|
| | 0% | 1% | 2% | 5% | 10% |
| Comparative Disp.Agent A | 0 | 1-2 | 1-2 | 3 | 3 |
| B | 0 | 1 | 2 | 3 | 3-4 |
| C | 0 | 2 | 2-3 | 3 | 3-4 |
| D | 0 | ½ | ½ | 3-4 | 3-4 |
| Dispersing Agent 1 | 0 | 1 | 1-2 | 2 | 3 |
| 2 | 0 | 0 | 0-1 | 2-3 | 3 |
| 3 | 0 | 0 | 0 | 0 | 0 |
| 4 | 0 | 0 | 0 | 0 | 0 |
| 5 | 0 | 0 | 0 | 1 | 1-2 |
| 6 | 0 | 0 | 0 | 0 | 1-2 |
| 7 | 0 | 0 | 1 | 2-3 | 3 |
| 8 | 0 | 0 | 1 | 1-2 | 3-4 |

The results in Tables 2 to 4 demonstrate the significant advantages for the dispersing agents of the present invention over the closest known dispersing agents in compatibility with a range of conventional paint resins.

Examples 33 to 36 and Comparative Examples 1 to 6

To evaluate the effect of the dispersing agents in accordance with the present invention on conventional thermosetting paint systems pigmented with a lead chrome pigment and a phthalocyanine pigment, an oven-drying alkyd-melamine paint system was employed. The alkyd resin used had the following formulation:

| | | | |
|---|---|---|---|
| Phthalic Anhydride | 42% | Fatty Acid | 40% |
| Glycerol | 18% | Xylene | 30% |

and the acid value of the solid portion was 7 mg KOH/g.

The resin is available as PLASTOKYD C—30 AX (PLASTOKYD is a registered trade mark).

In each of Examples 33 and 34 the pigment was dispersed in solvent in the presence of the dispersing agent. In Example 35 a fluidising agent was also added. In Example 36 the pigment was dispersed in the solvent in the presence of resin and the dispersing agent was added to the let-down solution.

The Comparative Examples were of 3 different types:

(i) Pigment was dispersed in the solvent in the presence of resin, in accordance with normal procedure in paint technology (Comparative Examples 1, 3 & 4);

(ii) Pigment was dispersed in the solvent in the presence of resin and two separate dispersing agents in accordance with UK 1,342,746 were added to the let-down solution (Comparative Examples 5 and 6);

(iii) Pigment was dispersed in solvent in the presence of a dispersing agent in accordance with UK 1,373,660 (Comparative Example 2).

All the paint formulations were prepared as a concentrated mill-base in a ball mill and in each case the mean particle diameter of the pigment, after milling, was below 5 microns. After milling the mill-base was let-down with further resin, cross-linking agent and solvent to produce a usable paint. The precise formulations of the mill-bases and let-down solutions are given in Tables 5 to 7 below, all quantities being expressed in grams weight.

Table 5

| Component | Comparative Example 1 | Comparative Example 2 | Example 33 | Example 34 |
|---|---|---|---|---|
| Millbase | | | | |
| Pigment Yellow 34 | 28.0 | 28.0 | 28.0 | 28.0 |
| PLASTOKYD C-30 AX | 6.54 | - | - | - |
| Dispersing Agent 1 | - | - | 1.4 | - |
| Dispersing Agent 3 | - | - | - | 1.4 |
| Comparative Agent C | - | 1.4 | - | - |
| Xylene | 14.66 | 9.2 | 9.2 | 9.2 |
| n-Butanol | 3.66 | | | |
| Let-down Solution | | | | |
| PLASTOKYD C-30 AX | 50.0 | 56.54 | 56.54 | 56.54 |
| BEETLE RE 615 | 27.5 | 27.5 | 27.5 | 27.5 |
| Xylene | 11.6 | 15.1 | 15.1 | 15.1 |
| n-Butanol | - | 3.66 | 3.66 | 3.66 |

Table 6

| Component | Comparative Example 3 | Example 35 |
|---|---|---|
| Millbase | | |
| Pigment Blue 15:2 | 6.1 | 6.1 |
| PLASTOKYD C-30 AX | 6.97 | - |
| Dispersing Agent 3 | - | 0.98 |
| Fluidising Agent 1 | - | 0.25 |
| Xylene | 13.53 | 10.41 |
| n-Butanol | 3.90 | 2.60 |
| Let-down Solution | | |
| PLASTOKYD C-30 AX | 54.2 | 59.77 |
| BEETLE RE 615 | 30.5 | 30.5 |
| Xylene | 6.8 | 11.39 |

Table 7

| Component | Comparative Example 4 | Comparative Example 5 | Comparative Example 6 | Example 36 |
|---|---|---|---|---|
| Millbase | | | | |
| Pigment Yellow 34 | 28.0 | 28.0 | 28.0 | 28.0 |
| PLASTOKYD C-30 AX | 6.54 | 6.54 | 6.54 | 6.54 |
| Xylene | 14.66 | 14.66 | 14.66 | 14.66 |
| n-Butanol | 3.66 | 3.66 | 3.66 | 3.66 |
| Let-down Solution | | | | |
| PLASTOKYD C-30 AX | 50.0 | 50.0 | 50.0 | 50.0 |
| BEETLE RE 615 | 27.5 | 27.5 | 27.5 | 27.5 |
| Comparative Agent A | - | 2.8 | - | |
| Comparative Agent D | - | - | 2.8 | |
| Dispersing Agent 1 | - | - | - | 2.8 |
| SOLVESSO 100 | 5.3 | 5.3 | 5.3 | 5.3 |
| Xylene | 9.1 | 6.3 | 6.3 | 6.3 |

A test panel was prepared for each of the paints identified in Tables 5 to 7 above and these were assessed for gloss and hardness. For the assessment of gloss, in accordance with ASTM D 523 (Specular gloss measured at an incidence angle of 20°), spray panels were prepared on anodised aluminium with an average dry paint film thickness of about 8 microns. For the assessment of hardness, in accordance with British Standard Test BS 3900: Part E 2, draw-down panels were prepared on anodised aluminium with an average dry paint film thickness of about 1.8 microns. The results of the assessments for gloss and hardness are shown in Table 8.

Table 8

| | Hardness BS 3900: Part E 2 | Gloss (20°) ASTM D 523 |
|---|---|---|
| Comparative Example 1 | 900 | 87 |
| Comparative Example 2 | 300 | 76 |
| Example 33 | 800 | 88 |
| Example 34 | 1000 | 88 |
| Comparative Example 3 | 1050 | - |
| Example 35 | 950 | - |
| Comparative Example 4 | 1200 | 92 |
| Comparative Example 5 | 400 | 90 |
| Comparative Example 6 | 700 | 92 |
| Example 36 | 1200 | 94 |

It will be observed that the paints of Examples 33 and 34 have similar properties to the paint of Comparative Example 1 (a conventional paint) whereas the paint of Comparative Example 2 (containing a known dispersing agent, Comparative Agent C from UK 1,373,660) is inferior in terms of both hardness and gloss. The paint of Example 35 has similar properties to Comparative Example 3 (a conventional paint). The paint of Example 36 has similar properties to the paint of Comp. Example 4 (a conventional paint) while the paints of Comp. Examples 5 and 6 (which contain known dispersing agents, Comparative Agents A and D from UK 1,342,746) are inferior in terms of hardness.

Example 37 and Comparative Examples 7 and 8

To further evaluate the effect of the dispersing agents in accordance with the present invention on conventional thermosetting paint systems pigmented with a lead chrome pigment, a thermosetting acrylic paint system was employed. The acrylic resin employed was SYNOCRIL 826S, and the crosslinking agent employed was BEETLE BE 683, a low viscosity n-butylated melamine formaldehyde resin supplied as a 75% solution in n-butanol.

All the paint formulations were prepared as a concentrated millbase in a ball mill, and in each case the mean particle diameter of the pigment, after milling, was below 5 microns. After milling the millbase was let-down with acrylic resin, cross-linking agent and solvent to produce a usable paint. The precise formulations of the millbases and let-down solutions are given in Table 9 below, all quantities being expressed in grams weight.

Table 9

| Component | Comparative Example 7 | Comparative Example 8 | Example 37 |
|---|---|---|---|
| Millbase | | | |
| Pigment Yellow 34 | 28.0 | 36.92 | 28.0 |
| Dispersing Agent 1 | - | - | 1.4 |
| Comparative Agent A | - | 1.84 | - |
| SYNOCRIL 826S | 7.63 | - | - |
| Xylene 12.21 | 12.13 | 9.2 | |
| n-butanol | 3.05 | - | - |
| Let-down Solution | | | |
| SYNOCRIL 826S | 46.33 | 68.08 | 51.63 |
| BEETLE BE 683 | 29.91 | 39.44 | 29.91 |
| Xylene | 8.77 | 23.31 | 17.72 |
| n-butanol | 2.13 | 2.81 | 2.13 |

A test panel was prepared for each of the paints identified in Table 9, and assessed for gloss and hardness, as described for Examples 33 to 36, and Comparative Examples 1 to 6. The results are shown in Table 10.

Table 10

| | Hardness BS 3900:Part E2 | Gloss (20°) ASTM D 523 |
|---|---|---|
| Comparative Example 7 | 1100 | 92 |
| Comparative Example 8 | 800 | 92 |
| Example 37 | 1100 | 92 |

It will be seen that the paint of Example 37 has similar properties to the paint of Comparative Example 7 (a conventional paint) whereas the paint of Comparative Example 8 (containing a known dispersing agent, Comparative Agent A, from UK 1,342,746) is inferior in terms of hardness.

## Claims

1. A compound of the formula:

$$B—([O—A—CO]_n—J)_m$$

wherein

A is alkylene or alkenylene containing from 8 to 25 carbon atoms with at least 4 carbon atoms between the oxygen atom and the carbonyl group;

m is 1 or 2;

n is an integer from 1 to 19;

B is $Y(—CO—)_m$ in which:

when m = 1, Y is alkyl, alkenyl, alkapolyenyl containing from 8 to 25 carbon atoms or arylalkyl or arylalkenyl containing up to 50 carbon atoms, and

when m = 2, Y is alkylene, alkenylene or alkapolyenylene containing up to 50 carbon atoms;

and

J is —OM in which M is H, $NH_4$ or a metal;

or

J is —ZR in which:

Z is —N(Q)—L— or —O—L— in which:

Q is H or $C_{1-25}$-alkyl; and

L is $C_{2-6}$-alkylene or $C_{2-6}$-hydroxyalkylene; and

R is an amino group or the salt thereof with an acid;

or

R is a sulphonic acid, phosphonic acid, sulphate or phosphate group, or the salt thereof with a metal or an amine;

provided that at least 30% of the total number of groups represented by A, B and J carry an epoxy group.

2. A compound according to Claim 1 wherein at least 30% of the groups represented by A carry an epoxy group.

3. A compound according to Claim 2 wherein at least 30% of the groups represented by A are 8,9-epoxy-11-n-heptyl-unadec-1,11-ylene.

4. A compound according to Claim 1 wherein at least 50% of the groups represented by A carry an epoxy group.

5. A compound according to any one of Claims 1 to 4 wherein the amino group or the salt thereof with an acid, represented by R is a primary, secondary or tertiary amino group or a substituted ammonium group of the formula:

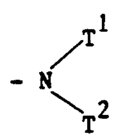

or

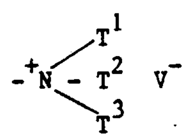

wherein $T^1$, $T^2$ & $T^3$ are each independently H, $C_{1-25}$-alkyl or $C_{3-8}$-cycloalkyl; and

V is the anion of an inorganic or organic acid or of an organic dye containing a $—SO_3H$ or —COOH group.

6. A compound according to any one of Claims 1 to 5 wherein the amino group R is a poly(alkyleneimine).

7. A compound according to Claim 6 wherein the amino group R is a poly(ethyleneimine) having a molecular weight in the range from 500 to 100,000.

8. A process for the preparation of a compound according to Claim 1 which comprises reacting an epoxidising agent with a compound of the formula:

$$B—([O—A—CO]_n—J)_m$$

wherein B, A and J are as previously defined, except that they are free from epoxy groups, and at least 30% of the total number of the groups B, A and J contain a carbon-carbon double bond.

9. A process for the preparation of a compound according to Claim 1 which comprises reacting a compound of the formula $B(—W)_m$ with a compound of the formula:

$$HO—A—CO—(O—A—CO)_{n-1}—J$$

wherein W is a group reactive with hydroxy groups, and A, B and J are as defined in claim 1.

10. A dispersible composition of a solid and from 1% to 50% by weight, based on the weight of the solid, of a compound of the formula:

$$B—([O—A—CO]_n—J)_m$$

wherein

A is alkylene or alkenylene containing from 8 to 25 carbon atoms with at least 4 carbon atoms between the oxygen atom and the carbonyl group;

m is 1 or 2;

n is an integer from 1 to 19;

B is $Y(—CO—)_m$ in which:

when m = 1, Y is alkyl, alkenyl, alkapolyenyl, or arylalkyl or arylalkenyl containing up to 50 carbon atoms, and

when m = 2, Y is alkylene, alkenylene or alkapolyenylene containing up to 50 carbon atoms;

and

J is —OM in which M is H, $NH_4$ or a metal;

or

J is —ZR in which:

Z is —N(Q)—L— or —O—L— in which:

Q is H or $C_{1-25}$-alkyl; and

L is $C_{2-6}$-alkylene or $C_{2-6}$-hydroxyalkylene; and

R is an amino group or the salt thereof with an acid;

or

R is a sulphonic acid, phosphonic acid, sulphate or phosphate group, or the salt thereof with a metal or an amine;

provided that at least 30% of the total number of groups represented by A, B and J carry an epoxy group.

11. A dispersion of from 5% to 85% by weight, based on the total weight of the dispersion, of a solid in an organic liquid containing from 1% to 50% by weight, based on the weight of the solid, of a compound of the formula:

$$B—([O—A—CO]_n—J)_m$$

wherein

A is alkylene or alkenylene containing from 8 to 25 carbon atoms with at least 4 carbon atoms between the oxygen atom and the carbonyl group;

m is 1 or 2;

n is an integer from 1 to 19;

B is $Y(—CO—)_m$ in which:

when m = 1, Y is alkyl, alkenyl, alkapolyenyl, arylalkyl or arylalkenyl containing up to 50 carbon atoms, and

when m = 2, Y is alkylene, alkenylene or alkapolyenylene containing up to 50 carbon atoms;

and

J is —OM in which M is H, $NH_4$ or a metal;

or

J is —ZR in which:

Z is —N(Q)—L— or —O—L— in which:

Q is H or $C_{1-25}$-alkyl; and

L is $C_{2-6}$-alkylene or $C_{2-6}$-hydroxyalkylene; and

R is an amino group or the salt thereof with an acid;

or

R is a sulphonic acid, phosphonic acid, sulphate or phosphate group, or the salt thereof with a metal or an amine;

provided that at least 30% of the total number of groups represented by A, B and J carry an epoxy group.

12. A dispersible composition or a dispersion according to Claim 10 or Claim 11 wherein the amino group or the salt thereof with an acid, represented by R in the definition of the dispersant, is a primary, secondary or tertiary amino group or a substituted ammonium group of the formula:

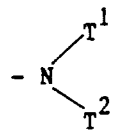 or 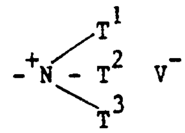

wherein $T^1$, $T^2$ & $T^3$ are each independently H, $C_{1-25}$-alkyl or $C_{3-8}$-cycloalkyl; and

V is the anion of an inorganic or organic acid or of an organic dye containing a $—SO_3H$ or —COOH group.

13. A dispersible composition or dispersion according to Claim 10 or Claim 11 wherein the solid is a pigment.

14. A dispersion according to Claim 11 or Claim 12 wherein the organic liquid is an aromatic hydrocarbon or an aliphatic or aromatic halogenated hydrocarbon or a mixture of this with an ester, an aliphatic alcohol or a ketone.

**Patentansprüche**

1. Verbindung der Formel

$$B—([O—A—CO]_n—J)_m$$

worin

A für Alkylen oder Alkenylen mit 8 bis 25 Kohlenstoffatomen steht, wobei mindestens 4 Kohlenstoffatome zwischen dem Sauerstoffatom und der Carbonylgruppe vorliegen;

m für 1 oder 2 steht;

n fur eine ganze Zahl von 1 bis 19 steht;

B für $Y(—CO—)_m$ steht, worin

wenn m = 1 Y Alkyl, Alkenyl, Alkapolyenyl mit 8 bis 25 Kohlenstoffatomen oder Arylalkyl oder Arylalkenyl mit bis zu 50 Kohlenstoffatomen bedeutet, und

wenn m = 2 Y Alkylen, Alkenylen oder Alkapolyenylen mit bis zu 50 Kohlenstoffatomen bedeutet; und

J für —OM steht, worin M H, $NH_n$ oder ein Metall bedeutet;

oder

J für —ZR steht, worin

Z —N(Q)—L— oder —O—L— bedeutet, worin

Q H oder $C_{1-25}$-Alkyl ist und

L $C_{2-6}$-Alkylen oder $C_{2-6}$-Hydroxyalkylen ist; und

R eine Aminogruppe oder ein Salz davon mit einer Säure bedeutet;

oder

R eine Sulfonsäure-, Phosphonsäure-, Sulfat- oder Phosphatgruppe oder ein Salz davon mit einem Metall oder einem Amin bedeutet;

mit der Maßgabe, daß mindestens 30% der durch A, B und J dargestellten Gruppen eine Epoxygruppe tragen.

2. Verbindung nach Anspruch 1, in welcher mindestens 30% der durchh A dargestellten Gruppen eine Epoxygruppe tragen.

3. Verbindung nach Anspruch 2, in welcher mindestens 30% der durch A dargestellten Gruppen 8,9-Epoxy-11-n-heptyl-unadec-1,11-ylene-Gruppen sind.

4. Verbindung nach Anspruch 1, worin mindestens 50% der durch A dargestellten Gruppen eine Epoxygruppe tragen.

5. Verbindung nach einem der Ansprüche 1 bis 4, in welcher die Aminogruppe oder das Salz davon mit einer Saüre, welche durch R dargestellt werden, eine primäre, sekundäre oder tertiäre Aminogruppe oder eine substituierte Ammoniumgruppe der Formel

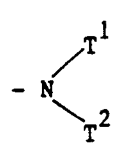 oder 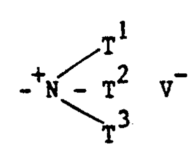 ist,

worin

$T^1$, $T^2$ und $T^3$ jeweils unabhängig voneinander für H, $C_{1-25}$-Alkyl oder $C_{3-8}$-Cycloalkyl stehen; und

V für das Anion einer anorganischen oder organischen Säure oder für einen organischen Farbstoff, der eine $—SO_3H$ oder —COOH-Gruppe enthält, steht.

6. Verbindung nach einem der Ansprüche 1 bis 5, in welcher die Aminogruppe R ein Poly(alkylenimin) ist.

7. Verbindung nach Anspruch 6, in welcher die Aminogruppe R ein Poly(ethylenimin) mit einem Molekulargewicht im Bereich von 500 bis 100000 ist.

8. Verfahren zur Herstellung einer Verbindung nach Anspruch 1, bei welchem ein Epoxidierungsmittel mit einer Verbindung der Formel

$$B—([O—A—CO]_n—J)_m$$

umgesetzt wird, wobei B, A und J die oben angegebenen Bedeutungen besitzen, außer daß sie frei von Epoxygruppen sind und daß mindestens 30% der Gesamtzahl der Gruppen A, B und J eine Kohlenstoff-Kohlenstoff-Doppelbindung enthalten.

9. Verfahren zur Herstellung einer Verbindung nach Anspruch 1, bei welchem eine Verbindung der Formel $B(—W)_m$ mit einer Verbindung der Formel

$$HO—A—CO—(O—A—CO)_{n-1}—J$$

umgesetzt wird, worin B eine mit Hydroxygruppen reaktive Gruppe ist und A, B und J die in Anspruch 1 angegebenen Definitionen besitzen.

10. Dispergierbare Zusammensetzung aus einem Feststoff und aus 1 bis 50 Gew.-%, bezogen auf das Gewicht des Feststoffs, einer Verbindung der Formel

$$B—([O—A—CO]_n—J)_m$$

worin

A für Alkylen oder Alkenylen mit 8 bis 25 Kohlenstoffatomen steht, wobei mindestens 4 Kohlenstoffatome zwischen dem Sauerstoffatom und der Carbonylgruppe vorliegen;

m für 1 oder 2 steht;

n fur eine ganze Zahl von 1 bis 19 steht;

B für $Y(—CO—)_m$ steht, worin

wenn m = 1 Y Alkyl, Alkenyl, Alkapolyenyl mit 8 bis 25 Kohlenstoffatomen oder Arylalkyl oder Arylalkenyl mit bis zu 50 Kohlenstoffatomen bedeutet, und

wenn m = 2 Y Alkylen, Alkenylen oder Alkapolyenylen mit bis zu 50 Kohlenstoffatomen bedeutet; und

J für —OM steht, worin M H, $NH_4$ oder ein Metall bedeutet;

oder

J für —ZR steht, worin

Z —N(Q)—L— oder —O—L— bedeutet, worin

Q H oder $C_{1-25}$-Alkyl ist und

L $C_{2-6}$-Alkylen oder $C_{2-6}$-Hydroxyalkylen ist; und

R eine Aminogruppe oder ein Salz davon mit einer Säure bedeutet;

oder

R eine Sulfonsäure-, Phosphonsäure-, Sulfat- oder Phosphatgruppe oder ein Salz davon mit einem Metall oder einem Amin bedeutet;

mit der Maßgabe, daß mindestens 30% der durch A, B und J dargestellten Gruppen eine Epoxygruppe tragen.

11. Dispersion aus 5 bis 85 Gew.-%, bezogen auf das Gesamtgewicht der Dispersion, eines Feststoffs in einer organischen Flüssigkeit, die 1 bis 50 Gew.-%, bezogen auf das Gewicht des Feststoffs, einer Verbindung der Formel enthält

$$B—([O—A—CO]_n—J)_m$$

worin

A für Alkylen oder Alkenylen mit 8 bis 25 Kohlenstoffatomen steht, wobei mindestens 4 Kohlenstoffatome zwischen dem Sauerstoffatom und der Carbonylgruppe vorliegen;

m für 1 oder 2 steht;

n fur eine ganze Zahl von 1 bis 19 steht;

B für $Y(—CO—)_m$ steht, worin

wenn m = 1 Y Alkyl, Alkenyl, Alkapolyenyl mit 8 bis 25 Kohlenstoffatomen oder Arylalkyl oder Arylalkenyl mit bis zu 50 Kohlenstoffatomen bedeutet, und

wenn m = 2 Y Alkylen, Alkenylen oder Alkapolyenylen mit bis zu 50 Kohlenstoffatomen bedeutet; und

J für —OM steht, worin M H, $NH_n$ oder ein Metall bedeutet;

oder

J für —ZR steht, worin

Z —N(Q)—L— oder —O—L— bedeutet, worin

Q H oder $C_{1-25}$-Alkyl ist und

L $C_{2-6}$-Alkylen oder $C_{2-6}$-Hydroxyalkylen ist; und

R eine Aminogruppe oder ein Salz davon mit einer Säure bedeutet;

oder

R eine Sulfonsäure-, Phosphonsäure-, Sulfat- oder Phosphatgruppe oder ein Salz davon mit einem Metall oder einem Amin bedeutet;

mit der Maßgabe, daß mindestens 30% der durch A, B und J dargestellten Gruppen eine Epoxygruppe tragen.

12. Dispergierbare Zusammensetzung oder Dispersion nach Anspruch 10 oder Anspruch 11, in welcher die Aminogruppe oder das Salz davon mit einer Säure, die durch R in der Definition des Dispergiermittels dargestellt werden, eine primäre, sekundäre oder tertiäre Aminogruppe oder eine substituierte Ammoniumgruppe der Formel

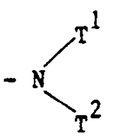

oder

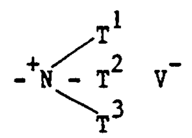

ist,

worin

$T^1$, $T^2$ und $T^3$ jeweils unabhängig voneinander für H, $C_{1-25}$-Alkyl oder $C_{3-8}$-Cycloalkyl stehen; und

V für das Anion einer anorganischen oder organischen Säure oder für einen organischen Farbstoff, der eine —$SO_3H$ oder —COOH-Gruppe enthält, steht.

13. Dispergierbare Zusammensetzung oder Dispersion nach Anspruch 10 oder 11, in welcher der Feststoff ein Pigment ist.

14. Dispersion nach Anspruch 11 oder 12, in welcher die organische Flüssigkeit ein aromatischer Kohlenwasserstoff oder ein aliphatischer oder aromatischer halogenierter Kohlenwasserstoff oder ein Gemisch derselben mit einem Ester, einem aliphatischen Alkohol oder einem Keton ist.

**Revendications**

1. Composé de formule:

$$B—([O—A—CO]_n—J)_m$$

dans laquelle

A est un groupe alkylène ou alcénylène contenant 8 à 25 atomes de carbone avec au moins 4 atomes de carbone entre l'atome d'oxygène et le groupe carbonyle;

m a la valeur 1 ou 2;

n est un nombre entier de 1 à 19;

B représente un groupe $Y(—CO—)_m$ dans lequel:

lorsque m est égal à 1, Y est un groupe alkyle, alcényle, alkapolyényle contenant 8 à 25 atomes de carbone ou arylalkyle ou arylalcényle contenant jusqu'à 50 atomes de carbone, et

lorsque m est égal à 2, Y est un groupe alkylène, alcénylène ou alkapolyénylène contenant jusqu'à 50 atomes de carbone;

et

J est un groupe —OH dans lequel M représente H, $NH_n$ ou un métal;

ou bien

J est un groupe —ZR dans lequel:

Z est un groupe —N(Q)—L— ou —O—L— dans lequel:

Q représente H ou un groupe alkyle en $C_1$ à $C_{25}$; et

L est un groupe alkylène en $C_2$ à $C_6$ ou hydroxyalkylène en $C_2$ à $C_6$; et

R est un groupe amino ou son sel avec un acide;

ou bien

R est un groupe acide sulfonique, acide phosphonique, sulfate ou phosphate, ou son sel avec un métal ou une amine;

sous réserve qu'au moins 30% du nombre total de groupes représentés par A, B et J portent un groupe époxy.

2. Composé suivant la revendication 1, dans lequel au moins 30% des groupes représentés par A portent un groupe époxy.

3. Composé suivant la revendication 2, dans lequel au moins 30% des groupes représentés par A sont des groupes 8,9-époxy-11-n-heptyl-undéc-1,11-ylène.

4. Composé suivant la revendication 1, dans lequel au moins 50% des groupes représentés par A portent un groupe époxy.

5. Composé suivant l'une quelconque des revendications 1 à 4, dans lequel le groupe amino ou son sel avec un acide, représenté par R, est un groupe amino primaire, secondaire ou tertiaire ou un groupe ammonium substitué de formule:

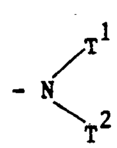

ou

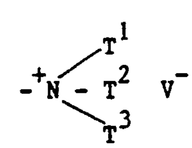

dans laquelle

$T^1$, $T^2$ & $T^3$ représentent chacun, indépendamment, H, un groupe alkyle en $C_1$ à $C_{25}$ ou un groupe cycloalkyle en $C_3$ à $C_8$; et

V est l'anion d'un acide inorganique ou organique ou d'un colorant organique contenant un groupe $—SO_3H$ ou —COOH.

6. Composé suivant l'une quelconque des revendications 1 à 5, dans lequel le groupe amino R est un groupe poly(alkylène-imine).

7. Composé suivant la revendication 6, dans lequel le groupe amino R est un groupe poly(éthylène-imine) ayant un poids moléculaire dans l'intervalle de 500 à 100 000.

8. Procédé de préparation d'un composé suivant la revendication 1, qui consiste à faire réagir un agent époxydant avec un composé de formule:

$$B—([O—A—CO]_n—J)_m$$

dans laquelle B, A et J sont tels que définis ci-dessus, excepté qu'ils sont dépourvus de groupes époxy, et au moins 30% du nombre total des groupes B, A et J contiennent une double liaison carbone à carbone.

9. Procédé de préparation d'un composé suivant la revendication 1, qui consiste à faire réagir un composé de formule $B(—W)_m$ avec un composé de formule:

$$HO—A—CO—(O—A—CO)_{n-1}—J$$

dans laquelle W est un groupe réactif avec des groupes hydroxy et A, B et J sont tels que définis dans la revendication 1.

10. Composition dispersible d'une matière solide et de 1 à 50% en poids, sur la base du poids de la matière solide, d'un composé de formule:

$$B—([O—A—CO]_n—J)_m$$

dans laquelle

A est un groupe alkylène ou alcénylène contenant 8 à 25 atomes de carbone avec au moins 4 atomes de carbone entre l'atome d'oxygène et le groupe carbonyle;

m a la valeur 1 ou 2;

n est un nombre entier de 1 à 19;

B représente un groupe $Y(—CO—)_m$ dans lequel:

lorsque m est égal à 1, Y est un groupe alkyle, alcényle, alkapolyényle, arylalkyle ou arylalcényle contenant jusqu'à 50 atomes de carbone, et

lorsque m est égal à 2, Y est un groupe alkylène, alcénylène ou alkapolyénylène contenant jusqu'à 50 atomes de carbone;

et

J est un groupe —OH dans lequel M représente H, $NH_4$ ou un métal;

ou bien

J est un groupe —ZR dans lequel:

Z est un groupe —N(Q)—L— ou —O—L— dans lequel:

Q représente H ou un groupe alkyle en $C_1$ à $C_{25}$; et

L est un groupe alkylène en $C_2$ à $C_6$ ou hydroxyalkylène en $C_2$ à $C_6$; et

R est un groupe amino ou son sel avec un acide;

ou bien

R est un groupe acide sulfonique, acide phosphonique, sulfate ou phosphate, ou son sel avec un métal ou une amine;

sous réserve qu'au moins 30% du nombre total de groupes représentés par A, B et J portent un groupe époxy.

11. Dispersion de 5 à 85% en poids, sur la base du poids total de la dispersion, d'une matière solide dans un liquide organique contenant 1 à 50% en poids, sur la base du poids de la matière solide, d'un composé de formule:

$$B—([O—A—CO]_n—J)_m$$

dans laquelle

A est un groupe alkylène ou alcénylène contenant 8 à 25 atomes de carbone avec au moins 4 atomes de carbone entre l'atome d'oxygène et le groupe carbonyle;

m a la valeur 1 ou 2;

n est un nombre entier de 1 à 19;

B représente un groupe $Y(—CO—)_m$ dans lequel:

lorsque m est égal à 1, Y est un groupe alkyle, alcényle, alkapolyényle, arylalkyle ou arylalcényle contenant jusqu'à 50 atomes de carbone, et

lorsque m est égal à 2, Y est un groupe alkylène, alcénylène ou alkapolyénylène contenant jusqu'à 50 atomes de carbone;

et

J est un groupe —OH dans lequel M représente H, $NH_4$ ou un métal;

ou bien

J est un groupe —ZR dans lequel:

Z est un groupe —N(Q)—L— ou —O—L— dans lequel:

Q représente H ou un groupe alkyle en $C_1$ à $C_{25}$; et

L est un groupe alkylène en $C_2$ à $C_6$ ou hydroxyalkylène en $C_2$ à $C_6$; et

R est un groupe amino ou son sel avec un acide;

ou bien

R est un groupe acide sulfonique, acide phosphonique, sulfate ou phosphate, ou son sel avec un métal ou une amine;

sous réserve qu'au moins 30% du nombre total de groupes représentés par A, B et J portent un groupe époxy.

12. Composition dispersible ou dispersion suivant la revendication 10 ou la revendication 11, dans laquelle le groupe amino ou son sel avec un acide, représenté par R dans la définition de l'agent dispersant, est un groupe amino primaire, secondaire ou tertiaire, ou un groupe ammonium substitué de formule:

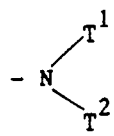

ou

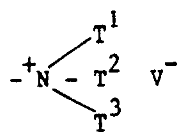

dans laquelle

$T^1$, $T^2$ & $T^3$ représentent chacun, indépendamment, H, un groupe alkyle en $C_1$ à $C_{25}$ ou un groupe cycloalkyle en $C_3$ à $C_8$; et

V est l'anion d'un acide inorganique ou organique ou d'un colorant organique contenant un groupe $—SO_3H$ ou —COOH.

13. Composition dispersible ou dispersion suivant la revendication 10 ou la revendication 11, dans laquelle la matière solide est un pigment.

14. Dispersion suivant la revendication 11 ou la revendication 12, dans laquelle le liquide organique est un hydrocarbure aromatique ou un hydrocarbure halogéné aliphatique ou aromatique ou leurs mélanges avec un ester, un alcool aliphatique ou une cétone.